# FASCICULE DE BREVET EUROPEEN

(11) **EP 0 460 979 B1**
(45) Date de publication et mention de la délivrance du brevet: **13.09.1995**
(21) Numéro de dépôt: 91401130.9
(22) Date de dépôt: 29.04.1991
(51) Int. Cl.: C12M 1/04, C12M 1/00, B01L 1/00, B01L 1/02, B25J 21/02, G21F 7/00, G21F 7/04

(54) **Procédé et installation de culture de germes anaérobies**
Verfahren und Vorrichtung zur Kultivierung von anaeroben Keimen
Process and apparatus for the culture of anaerobic microrganisms

(30) Priorité: 07.06.1990 FR 9007077
(43) Date de publication de la demande: 11.12.1991
(73) Titulaire: L'AIR LIQUIDE, SOCIETE ANONYME POUR L'ETUDE ET L'EXPLOITATION DES PROCEDES GEORGES CLAUDE, 75321 Paris Cédex 07 (FR)
(72) Inventeur: Willemot, Jean-Marie, F-92330 Sceaux (FR); Briend, Robert, F-78340 Les Clayes Sous Bois (FR)
(74) Mandataire: Le Moenner, Gabriel

(56) Documents cités:
- EP-A- 0 168 915
- GB-A- 2 174 714

## Description

La présente invention concerne un procédé et une installation de culture de germes anaérobies stricts dans un récipient, comportant l'étape préliminaire, avant incubation des germes, de créer dans le récipient contenant les germes et un catalyseur au palladium, une atmosphère gazeuse exempte d'oxygène et comprenant de l'anhydride carbonique et de l'hydrogène.

Les procédés connus d'obtention de conditions d'anaérobiose dans de tels récipients mettent en oeuvre des sachets générateurs de gaz sous la forme de comprimés effervescents libérant, dans le récipient, de l'anhydride carbonique et de l'hydrogène dans des conditions initiales en excès et créant dans le récipient une atmosphère gazeuse non contrôlée et dont la composition varie considérablement dans le temps. D'autre part, l'utilisation systématique de sachets avant chaque phase d'incubation rend cette technique relativement onéreuse.

Il a également été proposé une technique dite d'évacuation et de remplissage selon laquelle le récipient est soumis à un certain nombre (au minimum 6 et pouvant atteindre 12) de cycles successifs de mise sous vide partielle du récipient puis d'introduction d'un gaz constitué d'azote ou d'un mélange contenant de l'anhydride carbonique et de l'hydrogène. Cette technique se rélève également onéreuse dans la mesure où, outre la mise en oeuvre nécessaire d'une pompe à vide, les cycles successifs entraînent une consommation notable de gaz et prennent un temps certain.

La présente invention a pour objet de proposer un nouveau procédé obviant aux inconvénients des procédés connus, qui soit rapide à mettre en oeuvre, de prix de revient notablement réduit et garantissant des conditions d'anaérobiose contrôlées propices aux cultures bactériennes, et convenant de ce fait plus particulièrement pour les laboratoires d'analyses médicales indépendants, et les laboratoires de contrôle microbiologiques, notamment dans les industries agro-alimentaires ou de cosmétologie.

Pour ce faire, selon une caractéristique de l'invention, le procédé comprend les étapes de faire circuler dans le récipient, pendant un temps T₁, un mélange de gaz comportant environ 5 % d'anhydride carbonique, entre 3 et 10 % -typiquement entre 3,5 et 5% d'hydrogène, le complément étant de l'azote, puis d'isoler de façon étanche le récipient contenant le dit mélange gazeux.

De façon plus spécifique, pour un récipient ayant une capacité d'environ 3,5 litres, le débit de circulation du mélange gazeux est d'environ un litre par minute, le temps T₁ étant supérieur ou égal à 13 minutes, typiquement compris entre 14 et 17 minutes.

La présente invention a également pour objet une installation pour la mise en oeuvre de ce procédé, de conception simple et de faible coût et permettant une automatisation poussée de la phase de création d'atmosphère gazeuse contrôlée dans le récipient.

Pour ce faire, selon une autre caractéristique de l'invention, l'installation, du type comprenant : un récipient obturable de façon étanche par un couvercle muni d'un embout d'amenée de gaz à clapet anti-retour et d'un embout de sortie de gaz à clapet anti-retour, équipé d'une structure de rangement de conteneurs de germes et d'un logement pour un sachet de catalyseur, et une bouteille de mélange gazeux comprenant de l'anhydride carbonique et de l'hydrogène, connectable à l'embout d'amenée de gaz via un robinet détendeur de distribution, comprend en outre un ensemble de distribution et de commande comportant une ligne d'amenée de gaz connectable entre le robinet détendeur et l'embout d'amenée de gaz du récipient et munie d'une première électrovanne, et des moyens de commande pour actionner automatiquement la première électrovanne.

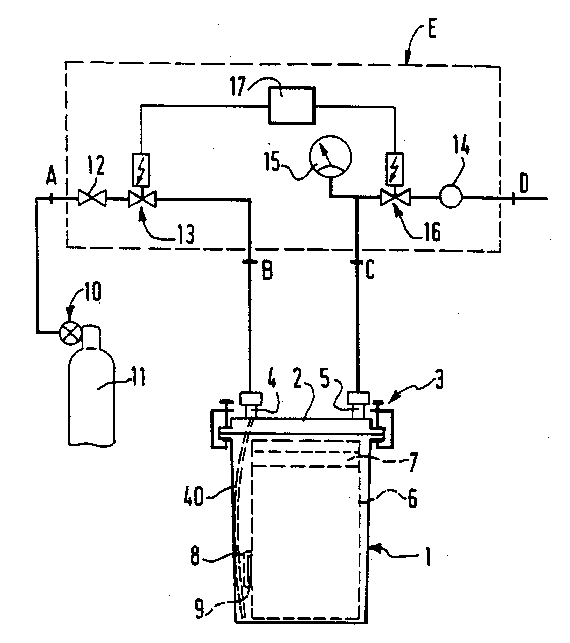

D'autres caractéristiques et avantages de la présente invention ressortiront de la description suivante d'un mode de réalisation, donné à titre illustratif mais nullement limitatif, faite en relation avec le dessin annexé sur lequel :
la figure unique représente schématiquement une installation selon l'invention.

On reconnait sur le dessin un récipient pour anaérobiose 1, classiquement appelé "jarre", hermétiquement obturable par un couvercle 2 verrouillable par au moins une pince 3, le couvercle étant muni d'un embout d'amenée de gaz 4 équipé d'un clapet anti-retour donnant passage uniquement dans la direction vers l'intérieur du récipient, et d'un embout de sortie de gaz 5 équipé d'un clapet anti-retour donnant passage uniquement dans le sens vers l'extérieur du récipient. Ce dernier comporte également une structure ou panier 6 pour le logement de conteneur de germes, typiquement des boîtes de Petri empilables 7 ou des tubes à essai. Selon l'invention le panier 6 est pourvu, au voisinage de sa base, d'un logement 8 pour un sachet 9 d'un catalyseur au palladium favorisant, à basse température, la combinaison de l'hydrogène et de l'oxygène et transformant donc en eau les traces d'oxygène résiduaire dans le récipient clos. L'embout d'amenée de gaz 4 est prolongé vers l'intérieur par un conduit flexible 40 s'étendant jusqu'au voisinage du fond du récipient 1.

L'installation selon l'invention comprend un ensemble de distribution et de commande comportant une ligne d'amenée de gaz A-B connectable entre un robinet détendeur 10 d'une bouteille de mélange gazeux 11 et l'embout 4. La ligne A-B comprend, de l'amont vers l'aval, un robinet de réglage de débit 12, et une première électrovanne 13. L'ensemble de distribution et de commande comporte en outre une ligne de retour de gaz C-D connectacle à l'embout 5 et débouchant en D dans l'atmosphère ou connectacle à une enceinte de récupération. La ligne C-D comprend, de l'amont vers l'aval, un manomètre 15, une seconde électrovanne 16 et un débimètre 14. L'ensemble est complété par un boîtier de commande électronique 17 couplé aux électrovannes 13 et 16 et, éventuellement à un manomètre 15 du type manostat, et incorporant un programme actionnant sélectivement les électrovannes 13 et 16, la première électrovanne 13 étant typiquement du type normalement fermé et la seconde électrovanne 16 étant typiquement du type normalement ouvert. L'ensemble de distribution et de commande est avantageusement intégré dans un boîtier E comprenant des raccords rapides au niveau des interfaces A à C.

Selon un aspect de l'invention, la bouteille 11 contient un mélange gazeux comportant environ 5 % d'anhydride carbonique, entre 3,5 et 5 %, typiquement 4,5 % d'hydrogène, le complément étant de l'azote.

La mise en oeuvre du procédé selon l'invention est la suivante : le récipient 1, contenant le catalyseur 9 et une série de conteneurs de germes 7, est fermé hermétiquement par le couvercle 2 au moyen des pinces 3. Les lignes A-B et D-C sont alors reliées, par exemple par des raccords de type à branchement rapide, aux embouts 4 et 5. Le robinet 10 de la bouteille 11 est ouvert et, le robinet 12 et les électrovannes 13 et 16 étant ouvertes, on fait circuler dans le récipient 1 le mélange gazeux contenu dans la bouteille 11 pendant un temps T₁ suffisant pour effectuer un balayage convenable de la capacité intérieure du récipient 1 et en chasser ainsi la quasi-totalité de l'air s'y trouvant initialement. Au bout du temps T₁, le boîtier de commande 17 actionne la fermeture de l'électrovanne 16 pendant un temps T₂ ce qui entraîne une pressurisation progressive du récipient 1, après quoi le boîtier 17 actionne la fermeture de l'électrovanne 13. Une alarme se déclenche alors, signalant la fin du conditionnement. Le récipient 1 étant ainsi isolé de la source de gaz sous pression 11 et de l'atmosphère, les lignes A-B et C-D sont déconnectées des embouts 4 et 5 et le récipient 1 ainsi conditionné peut être placé dans une étuve, typiquement pour une durée de 24 heures.

Pour un récipient 1 conventionnel, d'une capacité interne de 3,5 litres, le robinet 12 est réglé de façon à autoriser un débit de circulation de gaz d'environ un litre par minute pendant un temps T₁ non inférieur à 13 minutes, typiquement compris entre 14 et 17 minutes, de préférence pendant 16 minutes. On notera ici que, pour garantir de bonnes conditions d'anaérobiose, le temps et le débit de circulation doivent être très précisément appariés.

Le détendeur 10 délivre un mélange gazeux sous une faible pression typiquement de 2 x 10⁵ Pa (2 bars) absolus, pour donner, en raison des pertes de charge de la ligne A-B, au niveau de l'embout 4 une légère surpression, typiquement de l'ordre de 6 x 10⁴ Pa (0,6 bar). A la fin du temps T₁, l'électrovanne 16 est fermée pendant un temps T₂, de l'ordre de 50 à 60 secondes, suffisant pour créer dans le récipient 1 une surpression de sécurité entre 150 et 250 hPa, typiquement de l'ordre de 200 hPa, contre des risques de fuite au niveau du couvercle 2.

## Revendications

1. Procédé de culture de germes anaérobies stricts dans un récipient (1), comportant l'étape préliminaire, avant incubation, de créer dans le récipient contenant les germes (7) et un catalyseur au palladium (9), une atmosphère gazeuse comprenant de l'anhydride carbonique et de l'hydrogène, caractérisé en ce qu'il comprend les étapes suivantes :
- faire circuler dans le récipient (1), pendant un temps T₁, un mélange de gaz comportant environ 5 % d'anhydride carbonique, entre 3 et 10 % d'hydrogène, le complément étant de l'azote ;
- puis d'isoler de façon étanche le récipient (1) contenant le dit mélange gazeux.

2. Procédé selon la revendication 1, caractérisé en ce que le mélange gazeux comprend entre 3,5 et 5 % d'hydrogène.

3. Procédé selon la revendication 2, caractérisé en ce que, pour un récipient (1) de capacité d'environ 3,5 litres, le débit de circulation du mélange gazeux est d'environ un litre par minute, le temps T₁ étant supérieur ou égal à 13 minutes.

4. Procédé selon la revendication 3, caractérisé en ce que le temps T₁ est compris entre 14 et 17 minutes.

5. Procédé selon l'une des revendications précédentes, caractérisé en ce qu'il comprend l'étape, avant fermeture étanche du récipient (1), de créer dans ce dernier une surpression du dit mélange gazeux comprise entre 150 et 250 hPa.

6. Installation pour la mise en oeuvre du procédé selon l'une des revendications précédentes, comprenant : un récipient (1) obturable de façon étanche par un couvercle (2) muni d'un embout d'amenée de gaz (4) à clapet anti-retour et d'un embout (5) de sortie de gaz à clapet anti-retour, équipé d'une structure (6) de rangement de conteneurs de germes (7) et d'un logement (8) pour un sachet de catalyseur (9), et une bouteille (11) de mélange gazeux comprenant de l'anhydride carbonique et de l'hydrogène, connectacle à l'embout d'amenée de gaz (4) via un robinet détendeur (10), caractérisé en ce qu'elle comprend un ensemble de distribution et de commande (E) comportant :
- une ligne d'amenée de gaz (A-B) connectacle entre le robinet détendeur (10) et l'embout (4) d'amenée de gaz du récipient et muni d'une première électrovanne (13) ; et
- des moyens de commande (17) pour actionner automatiquement la première électrovanne (13).

7. Installation selon la revendication 6, caractérisée en ce que l'ensemble de distribution et de commande (E) comprend une ligne de retour de gaz (C-D) connectacle à l'embout (5) de sortie de gaz du récipient (1) et munie d'une seconde électrovanne (16) actionnable automatiquement par les moyens de commande (17).

8. Installation selon la revendication 7, caractérisée en ce que la ligne de retour de gaz (C-D) est munie de moyens de contrôle de pression (15) et de débit (14).

9. Installation selon l'une des revendications 6 à 8, caractérisée en ce que l'embout (4) d'amenée de gaz du récipient (1) est relié à une conduite d'amenée (40) s'étendant dans le récipient (1) jusqu'au voisinage du fond de celui-ci, le logement (8) du catalyseur (9) étant prévu dans la partie inférieure du récipient (1).

10. Installation selon l'une des revendications 6 à 9, caractérisée en ce que la bouteille (11) contient un mélange gazeux ternaire, constitué de 5 % d'anhydride carbonique, et d'entre 3,5 et 5 % d'hydrogène, le complément étant de l'azote.

## Claims

1. Method for growing strict anaerobic germs in a receptacle (1), including the primary step, before incubation, of creating, in the receptacle containing the germs (7) and a palladium catalyst (9), a gaseous atmosphere comprising carbon dioxide and hydrogen, characterised in that it comprises the following steps:
- circulating in the receptacle (1), for a time T₁, a mixture of gases including approximately 5% carbon dioxide and between 3 and 10% hydrogen, the remainder being nitrogen;
- then isolating, in a gastight manner, the receptacle (1) containing the said gaseous mixture.

2. Method according to Claim 1, characterised in that the gaseous mixture comprises between 3.5 and 5% hydrogen.

3. Method according to Claim 2, characterised in that, for a receptacle (1) having a capacity of approximately 3.5 litres, the circulation rate of the gaseous mixture is approximately one litre per minute, the time T₁ being greater than or equal to 13 minutes.

4. Method according to Claim 3, characterised in that the time T₁ is between 14 and 17 minutes.

5. Method according to one of the preceding Claims, characterised in that it comprises the step, before the gastight closure of the receptacle (1), of creating in the latter a pressure of the said gaseous mixture of between 150 and 250 hPa.

6. Installation for implementing the method according to one of the preceding claims, comprising: a receptacle (1) able to be closed in a gastight manner by a cover (2) provided with a gas supply connector (4) with a non-return valve and a gas outlet connector (5) with a non-return valve, equipped with a structure (6) for storing containers of germs (7) and a housing (8) for a catalyst sachet (9) and a bottle (11) of gaseous mixture comprising carbon dioxide and hydrogen, connectable to the gas supply connector (4) via a pressure-reducing valve (10), characterised in that it comprises a distribution and control unit (E) including:
- a gas supply line (A-B) connectable between the pressure-reducing valve (10) and the gas supply connector (4) of the receptacle and provided with a first solenoid valve (13); and
- control means (17) for automatically actuating the first solenoid valve (13).

7. Installation according to Claim 6, characterised in that the distribution and control unit (E) comprises a gas return line (C-D) connectable to the gas outlet connector (5) of the receptacle (1) and provided with a second solenoid valve (16) able to be actuated automatically by the control means (17).

8. Installation according to Claim 7, characterised in that the gas return line (C-D) is provided with pressure (15) and flow-rate (14) control means.

9. Installation according to one of Claims 6 to 8, characterised in that the gas supply connector (4) of the receptacle (1) is connected to a supply pipe (40) extending into the receptacle (1) close to the bottom of the latter, the housing (8) for the catalyst (9) being provided in the lower part of the receptacle (1).

10. Installation according to one of Claims 6 to 9, characterised in that the bottle (11) contains a ternary gaseous mixture consisting of 5% carbon dioxide and between 3.5 and 5% hydrogen, the remainder being nitrogen.

## Patentansprüche

1. Verfahren zur Kultivierung strikter anaerober Keime in einem Behälter (1), wobei in einem vorab durchgeführten Schritt vor der Inkubation in dem die Keime (7) und einen Palladium-Katalysator (9) enthaltenden Behälter eine Gasatmosphäre erzeugt wird, die Kohlensäureanhydrid und Wasserstoff enthält, dadurch gekennzeichnet, daß das Verfahren die folgenden Schritte aufweist:
- Umwälzen eines ungefähr 5% Kohlensäureanhydrid, zwischen 3 und 10 % Wasserstoff und als Rest Stickstoff enthaltenden Gasgemisches in dem Behälter (1) während einer Zeitdauer T₁;
- daraufhin abdichtendes Isolieren des das Gasgemisch enthaltenen Behälters (1).

2. Verfahren nach Anspruch 1, dadurch gekennzeichnet, daß das Gasgemisch zwischen 3,5 und 5 % Wasserstoff enhält.

3. Verfahren nach Anspruch 2, dadurch gekennzeichnet, daß für einen Behälter (1) mit einem Fassungsvermögen von ungefähr 3,5 Litern der umgewälzte Durchfluß des Gasgemisches ungefähr 1 Liter pro Minute ist, wobei die Zeit T₁ größer oder gleich 13 Minuten ist.

4. Verfahren nach Anspruch 3, dadurch gekennzeichnet, daß die Zeit T₁ zwischen 14 und 17 Minuten ist.

5. Verfahren nach einem der vorhergehenden Ansprüche, dadurch gekennzeichnet, daß es einen Schritt aufweist, um vor dem abdichtenden Absperren des Behälters (1) in ihm einen Überdruck des Gasgemisches zwischen 150 und 250 hPa zu erzeugen.

6. Vorrichtung zur Durchführung des Verfahrens nach einem der vorhergehenden Ansprüche, mit: einem Gefäß (1), das mittels eines Deckels (2) abdichtend verschließbar ist, der mit einem Gaseinlaßstutzen (4) mit Rückschlagklappe und einem Gasauslaßstutzen (5) mit Rückschlagklappe versehen ist, wobei das Gefäß mit einer Struktur (6) zur Anordnung von Behältern mit Keimen (7) und einem Sitz (8) für einen Katalysatorbeutel (9) ausgestattet ist, und einer Flasche (11) für Gasgemisch mit Kohlensäureanhydrid und Wasserstoff, die mit dem Gaseinlaßstutzen (4) über einen Druckminderventil (10) verbindbar ist, dadurch gekennzeichnet, daß die Vorrichtung eine Einheit zur Verteilung und Steuerung (E) aufweist, mit:
- einer Gaszufuhrleitung (A-B), die zwischen dem Druckminderventil (10) und dem Gaseinlaßstutzen (4) des Behälters angeschlossen werden kann und mit einem ersten Magnetventil (13) versehen ist; und
- einer Steuerungseinrichtung (17) zur automatischen Betätigung des ersten Magenetventils (13).

7. Vorrichtung nach Anspruch 6, dadurch gekennzeichnet, daß die Einheit zur Verteilung und Steuerung (E) eine Gasrücklaufleitung (C-D) aufweist, die mit dem Gasauslaßstutzen (5) des Gefäßes (1) verbunden werden kann und mit einem zweiten Magnetventil (16) zu versehen ist, das mittels der Steuereinrichtung (17) automatisch betätigt werden kann.

8. Vorrichtung nach Anspruch 7, dadurch gekennzeichnet, daß die Gasrücklaufleitung (C-D) mit Einrichtungen zur Überwachung des Drucks (15) und des Durchflusses (14) versehen ist.

9. Vorrichtung nach einem der Ansprüche 6 bis 8, dadurch gekennzeichnet, daß der Gaseinlaßstutzen (4) des Gefäßes (1) mit einer Gaszufuhrleitung (40) verbunden ist, die sich in dem Gefäß (1) bis in die Nähe seines Bodens erstreckt, wobei der Sitz (8) des Katalysators (9) in dem unteren Teil des Gefäßes (1) vorgesehen ist.

10. Vorrichtung nach einem der Ansprüche 6 bis 9, dadurch gekennzeichnet, daß die Flasche (11) ein ternäres Gasgemisch enthält, das aus 5 % Kohlensäureanhydrid und aus 3,5 bis 5 % Wasserstoff besteht, wobei der Rest Stickstoff ist.
